# EUROPEAN PATENT APPLICATION

(11) **EP 2 982 386 A1**
(43) Date of publication of application: **10.02.2016**
(21) Application number: 14179789.4
(22) Date of filing: 05.08.2014
(51) Int. Cl.: A61L 27/14, A61L 27/54, C08L 83/04

(54) **Breast implant comprising a nitric oxide releasing material**

(71) Applicant: Schrank, Christian, 82418 Murnau (DE); Mehler, Thomas, 82418 Murnau (DE)
(72) Inventor: Schrank, Christian, 82418 Murnau (DE); Mehler, Thomas, 82418 Murnau (DE)
(74) Representative: Kalhammer, Georg

(57) **Abstract**

The present invention relates to a breast implant comprising a flexible shell having a non-solid filling, said filling comprising a nitric oxide releasing material, wherein said flexible shell is permeable for nitric oxide. The invention further pertains to the use of such implant in plastic surgery.

## Description

### BACKGROUND OF THE INVENTION

Breast augmentation and augmentation mammoplasty are plastic surgery terms for the breast-implant and the fat-graft mammoplasty approaches used to increase the size, change the shape, and alter the texture of the breasts of a woman. As a primary reconstruction, augmentation mammoplasty is applied to effect a post-mastectomy breast reconstruction, the repair of the chest wound consequent to the removal of a cancerous breast; to correct congenital defects of the breast(s); and to correct congenital defects of the chest wall. As an elective, cosmetic surgery, primary augmentation changes the aesthetics - of size, shape, and texture - of healthy breasts.

The surgical implantation approach effects the global augmentation of the breast hemisphere using a breast implant, either an implant filled with saline-solution, or an implant filled with silicone-gel; moreover, the surgical augmentation approach can include the application of transplanted autologous skin flaps harvested from the woman's body.

The plastic surgical emplacement of breast-implant devices, either for breast reconstruction or for aesthetic purpose, presents the same health risks common to surgery, such as adverse reaction to anesthesia, hematoma (post-operative bleeding), seroma (fluid accumulation), incision-site breakdown (wound infection).

The human body's immune response to a surgically installed foreign object, e.g. a breast implant, is to encapsulate it with scar tissue capsules of tightly woven collagen fibers, in order to maintain the integrity of the body by isolating the foreign object, and so tolerate its presence. Capsular contracture - which should be distinguished from normal capsular tissue - occurs when the collagen-fiber capsule thickens and compresses the breast implant; it is a painful complication that might distort either the breast implant, or the breast, or both. The exact cause of capsular contracture is unknown, but the common incidence factors include bacterial contamination, device-shell rupture, filler leakage, and hematoma/seroma. The surgical implantation procedures that have reduced the incidence of capsular contracture include submuscular emplacement, the use of breast implants with a textured surface and polyurethane-coated implants; limited pre-operative handling of the implants, limited contact with the chest skin of the implant pocket before the emplacement of the breast implant, and irrigation of the recipient site with triple-antibiotic solutions. Further measures aiming to reduce the risk of capsular contracture include changing gloves, interim disinfection of chest skin, use of iodine/iodine-like solutions and use of new sterile coverings around the site of surgical interventions.

The correction of capsular contracture might require an open capsulectomy (surgical release) of the collagen-fiber capsule, or the removal, and possible replacement, of the breast implant. Furthermore, in treating capsular contracture, the closed capsulotomy (disruption via external manipulation) once was a technique for treating capsules, but now is rather a discouraged technique, because it can rupture the breast implant.

Despite the above-mentioned attempts to avoid capsular contracture, it is still one of the most common complications after breast implantation which is detected often only years after the initial surgery. As revision surgery is burdensome to the patients and has additional risks and costs, there is a need for means and methods to reduce bacterial infection and capsular contracture in breast augmentation using breast implants.

Nablo et al. (2005) Biomaterials 26, 6984-6990 report that implant-associated infections may be inhibited via nitric oxide release. They suggest that NO-releasing coatings may reduce the incidence of biomaterial-associated infection. Nablo et al. did not investigate implants comprising a shell and a non-solid filling. Furthermore, Nablo et al. applied the NO-releasing material only to the surface of the silicone rubber.

Hetrick et al. (2007) Biomaterials 28(31), 4571-4580 describe reduced foreign body response at nitric oxide-releasing subcutaneous implants. Solid silicone elastomer blocks were coated with diazeniumdiolate-modified xerogel polymers capable of releasing NO. Hetrick et al. did not investigate implants comprising a shell and a non-solid filling. Furthermore, Hetrick et al. applied the NO-releasing material only to the surface of the silicone elastomer blocks.

Coated implants have the disadvantage that they initially release a relatively high amount of nitric oxide, whereas there is no continuous release of NO over long periods of time, e.g. over months or even years.

### SUMMARY OF THE INVENTION

The inventors of this application surprisingly found that the above disadvantages can be reduced or avoided if the NO-releasing material is contained in the filling of the breast implant. This allows a continuous release of NO over long periods of time, without an initial burst phase of NO. The present invention therefore *inter alia* relates to the following embodiments (1) to (19):
(1) A breast implant comprising a flexible shell having a non-solid filling, said filling comprising a nitric oxide releasing material, wherein said flexible shell is permeable for nitric oxide.
(2) The breast implant of item (1), wherein said filling further comprises a silicone gel.
(3) The breast implant of item (1) or (2), wherein the nitric oxide releasing material is uniformly dispersed in said filling.
(4) The breast implant of any one of the preceding items, wherein the nitric oxide releasing material comprises or consists of a compound selected from the group consisting of organic nitrates, organic nitrites, metal-NO-complexes, N-nitrosamines, N-hydroxyl nitrosamines, nitrosimines, nitrosothiols, C-nitroso compounds, diazetine dioxides, furoxans, benzofuroxans, oxatriazole-5-imines, syndonimines, oxims, hydroxyamines, guanidines, N-hydroxyguanidines, hydroxyurea, hydroxamic acids, and nitroarenes.
(5) The breast implant of any one of the preceding items, wherein the nitric oxide is released from the breast implant for a period of at least one month, preferably for at least one year.
(6) The breast implant of any one of the preceding items, wherein the nitric oxide is released from the breast implant at a rate of from 1*10⁻¹² mole*min⁻¹*cm⁻² to 1*10⁻¹⁰ mole*min⁻¹*cm⁻²
(7) The breast implant of any one of the preceding items, wherein the flexible shell comprises a polysiloxane.
(8) The breast implant of any one of the preceding items, wherein the flexible shell comprises or consists of a material selected from the group consisting of polydimethylsiloxane, polydiphenylsiloxane, and combinations thereof.
(9) The breast implant of any one of the preceding items, wherein the flexible shell is coated with a nitric oxide releasing material.
(10) A breast implant comprising a nitric oxide releasing material for use in a method of breast augmentation, wherein said method comprises implanting the breast implant into a patient's breast, wherein said patient is resistant to at least one antibiotic.
(11) A breast implant comprising a nitric oxide releasing material for use in a method of breast augmentation, wherein said method comprises implanting the breast implant into a patient's breast without administering antibiotics to the patient.
(12) A breast implant comprising a nitric oxide releasing material for use in the prevention of bacterial infection during breast augmentation surgery.
(13) The breast implant for use according to item (12), wherein said bacterial infection is infection with multi-drug resistant bacteria.
(14) The breast implant for use according to any one of items (10) to (13), wherein said breast implant is a breast implant as defined in any one of items (1) to (9).
(15) A breast implant as defined in any one of items (1) to (9) for use in a method of breast augmentation.
(16) A breast implant as defined in any one of items (1) to (9) for use in a method of preventing bacterial infection during or after plastic surgery.
(17) The breast implant for use according to item (16), wherein said bacterial infection is infection with multi-drug resistant bacteria.
(18) A breast implant as defined in any one of items (1) to (9) for preventing the formation of capsular contracture upon breast augmentation surgery.
(19) A method of breast surgery, comprising inserting an implant according to any one of items (1) to (9) into a patient's breast tissue.

### BRIEF DESCRIPTION OF THE DRAWINGS

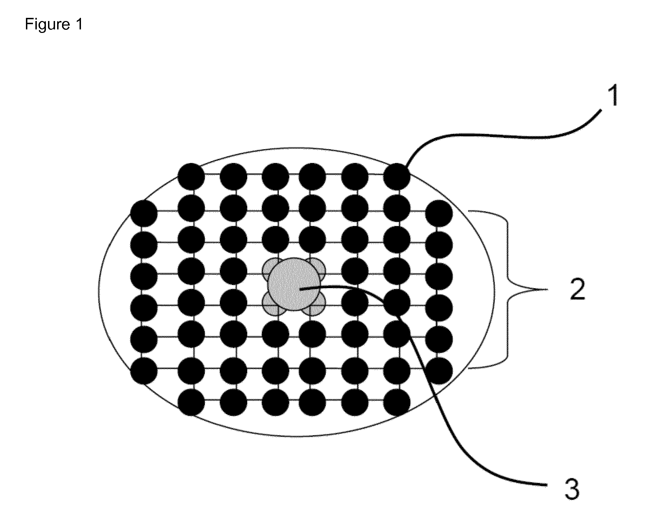

Figure 1 schematically depicts the structure of a nitric oxide releasing material that may be used in accordance with this invention. Multiple nitric oxide donor molecules (1) are linked with each other or embedded in a polymer matrix and/or a scaffold structure, thereby forming layers (2). The NO releasing polymer may have a core (3), especially in the case of dendrimer structures. The layers may have a three-dimensional spherical form around the core. The core may be a part of the polymer or even be absent. The NO-releasing materials may be manufactured by doping of low molecular weight NO donors, covalent tethering of NO donor functionalities to the polymer backbone, or doping of NO-releasing macromolecular scaffolds.

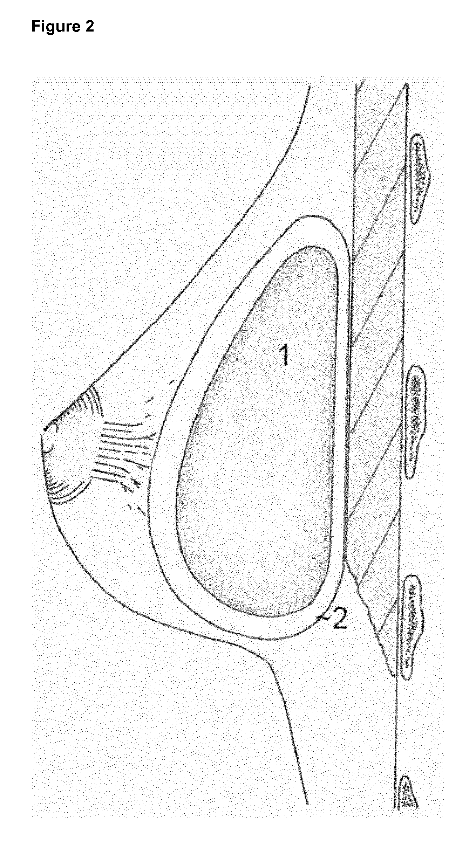

Figure 2 depicts a prior art breast implant (1) after implantation into a patient's breast. Following the surgical implantation, the surrounding tissue will form a capsule around the implant. A frequent undesired effect of the implantation is the formation of capsular contracture due to foreign body fibrosis (2).

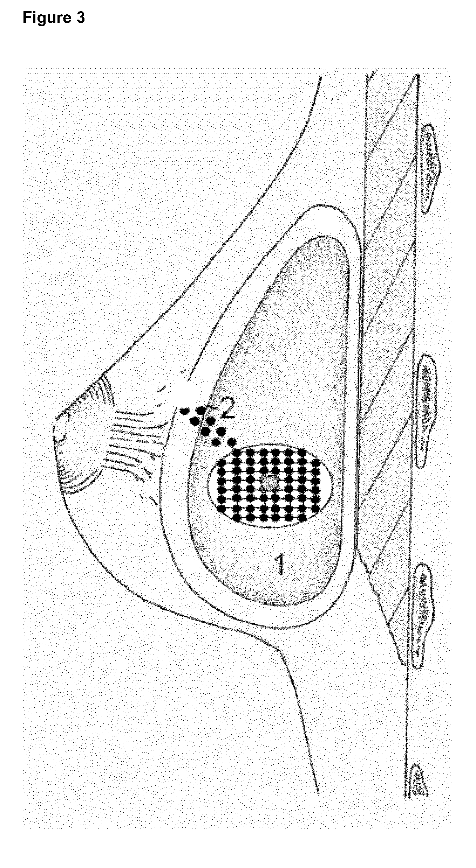

Figure 3 schematically depicts the effect of a breast implant of the present invention (1) after implantation into a patient's breast. The breast implant is a nitric oxide-charged implant (1) comprising NO-releasing polymers within its filling. The implant continuously releases nitric oxide into the surrounding tissue and thereby prevents capsular contracture (2).

### DETAILED DESCRIPTION

The present invention relates to a breast implant comprising a flexible shell having a filling, said filling comprising a nitric oxide releasing material, wherein said flexible shell is permeable for NO.

The shell of the breast implant may be made of any suitable pharmaceutically acceptable or medical grade material, provided it is permeable for NO. Preferably, the shell material comprises or consists of a silicone rubber. The silicone rubber may be made of polysiloxane(s), such as polydimethylsiloxane and polydiphenylsiloxane. In one embodiment, the shell comprises or consists of a polysiloxane. In preferred embodiments the shell comprises or consists of a material selected from the group consisting of polydimethylsiloxane, polydiphenylsiloxane, and combinations thereof.

In another embodiment, the shell comprises or consists of polyurethane.

The shell of the implant may be coated with a material selected from the group consisting of silicone, polyurethane, xero-gels, titan, and combinations thereof. The shell typically has a thickness in the range from 0.01 mm to 15 mm, preferably from 0.5 to 5 mm, for the NO to diffuse. The shell typically consists of multiples layers, e.g. of 2 to 10, preferably of 3 to 9, more preferably of 4 to 8, most preferably of 5 to 7 (e.g. 5, 6 or 7) layers.

The shell may be coated with an NO-releasing material, e.g. with a coating as described in US 2014/0017121 A1, US 5,676,963 or Riccio et al. (2012) ACS Appl Mater Interfaces 4(2): 796-804. For example, N-Acetyl Penicillamine Propyltrimethoxysilane (NAPTMS) and xerogels derived therefrom can be synthesized as described in Riccio et al. Total NO storage may range from 0.1 - 10 µmol/cm² based on the NAPTMS concentration and xerogel coating thickness, which is 0.001 mm to 100 mm.

The shell may comprise a plurality of layers, e.g. a base layer, an active layer, and a top layer. The base layer may comprise or consist of silicone resin(s); the active layer may comprise a silicone resin, N-(6-aminohexyl) aminopropyl trimethoxysiloxane (DACA-6), and DACA-6 of potassium tetrakis(4-chlorophenyl) borate; and/or the top layer may comprise silicone resin.

The filling of the implant typically comprises saline or a silicone gel.

A saline-filled breast implant has a shell which is inflated to the desired size with sterile isotonic saline. Saline-filled breast implants may vary in shell surface, shape, profile, volume, and shell thickness. The sterile saline used as a filler material preferably follows United States Pharmacopeia (USP) standards for Normal Physiological Saline (injection grade), which has a concentration of 0.15 M and a pH of 7.2-7.4.

The saline-filled breast implant may have one of the following designs:
- a fixed volume implant with a single lumen that is intraoperatively filled with the entire volume of saline via a valve;
- an adjustable volume implant with a single lumen that is intraoperatively filled with saline via a valve and has the potential for further postoperative adjustment of the saline volume;
- a prefilled saline implant.

All three designs are within the scope of the present invention.

A silicone gel-filled breast implant has a shell, which is filled with a fixed amount of silicone gel. Silicone gel-filled breast implants may vary in shell surface, shape, profile, volume, and shell thickness. The silicone gel-filled breast implant may have one of the following designs:
- a fixed volume implant with a single lumen containing a fixed amount of silicone gel
- an inflatable double lumen implant with the inner lumen containing a fixed amount of silicone gel and the outer lumen designed with a valve for filling with saline intraoperatively
- an inflatable double lumen implant with the outer lumen containing a fixed amount of silicone gel and the inner lumen designed with a valve for filling with saline intraoperatively, with the potential for postoperative adjustment of saline volume.
- A double lumen implant with the inner lumen containing a first silicone gel, and the outer lumen containing a second silicone gel, wherein the first silicone gel is harder than the second silicone gel, i.e. the second silicone gel is softer than the first silicone gel. This means that the inner lumen can better maintain a given form which leads to better projection of the imlant.

All four designs are within the scope of the present invention.

In certain embodiments, the filling is neither saline nor silicone gel. Instead, such an alternative breast implant typically has a silicone rubber shell with a filler other than saline or silicone gel. The filler material may or may not be a gel. However, an alternative breast implant may also have an alternative shell made from a material other than silicone rubber.

In accordance with this invention, the filling comprises a NO-releasing material. The NO-releasing materials may comprise or consist of one or more NO-releasing compounds. NO-releasing compounds include, but are not limited to, organic nitrates, organic nitrites, metal-NO-complexes, N-nitrosamines, N-hydroxyl nitrosamines, nitrosimines, nitrosothiols, C-nitroso compounds, diazetine dioxides, furoxans, benzofuroxans, oxatriazole-5-imines, syndonimines, oxims, hydroxyamines, guanidines, N-hydroxyguanidines, hydroxyurea, hydroxamic acids, and nitroarenes. NO-releasing compounds are described in "Nitric Oxide Donors", edited by Peng, Cai and Taniguchi, 2005, Wiley-VCH (ISBN-13: 978-3-527-31015-9) and the references cited therein. The disclosure of Peng et al. and of the references cited therein are incorporated by reference herein.

N-diazeniumdiolates (1-amino-substitutied diazen-1-ium-1,2-diolate) and S-nitrosothiols represent the two most diverse NO donor classes. N-diazeniumdiolates form on secondary amines under high pressures of NO in the presence of base. Upon proton-initiated degradation (e.g., in water), the NO donor releases two molecules of NO per secondary amine. S-nitrosothiols are an endogenous NO carrier but may be synthesized on free thiol groups upon exposure to a nitrosating agent (e.g., nitrous acid). Nitric oxide release of the sulfur-bound NO may be initiated by heat, light, or exposure to copper ion. Additionally, transnitrosation may occur resulting in the transfer of the S-nitrosothiol group to another free thiol group. Both of these NO donors spontaneously release NO without the need of other agents (e.g., enzymes). For example, small molecular weight NO donors that have been synthesized and used in biological studies include (Z)-1-[N-(3-aminopropyl)-N-(n-propyl)amino]diazen-1-ium-1,2-diolate (PAPA/NO), 1-[N-(aminoethyl)-N-(2-ammonioethyl)amino]diazen-1-ium-1,2-diolate (DETA/NO), S-nitrosoglutathione (GSNO), and S-nitroso-N-acetylpenicillamine (SNAP).

Metal nitrosyl compounds represent another class of compounds used to generate NO in biological studies. Two examples of metal nitrosyls are sodium nitroprusside (Na₂[Fe(CN)₅NO], SNP) and potassium nitrosylpentachlororuthenate (K₂[Ru(NO)Cl₅], NPR), with SNP being the most common. Metal nitrosyls are capable of releasing NO enzymatically, non-enzymatically when in the presence of vascular tissue and reducing agents (e.g., ascorbic acid), or by light irradiation.

N-diazeniumdiolates are most preferred NO-releasing compounds to be used in accordance with this invention. They may be incorporated into high molecular weight materials or macromolecular scaffolds in order to optimize the release of nitrix oxide. Macromolecular scaffolds have been reviewed in Riccio and Schoenfisch (2012) Chem. Soc. Rev. 41(10) 3731-3741, the content of which is incorporated herein by reference.

The NO-releasing material comprises or consists of at least one NO-releasing compound. In certain embodiments, the NO-releasing material is a polymer which comprises NO-releasing compounds. Preferred NO-releasing materials include:
- NO-releasing xerogels
   (Hetrick and Schoenfisch (2006) Chem. Soc. Rev. 35:780-789; Nablo et al.(2005) Biomaterials 26:6984-6990; Riccio et al. (2009) Biomaterials 30:4494-4502);
   in particular NO-releasing tertiary S-nitrosothiol-modified xerogels
   (Riccio et al. (2012) ACS Appl Mater Interfaces 4(2) 796-804)
- NO-releasing silica nanoparticles
   (Hetrick et al. (2009) Biomaterials 30(14) 2782-2789; Shin JH et al.(2007) J. Am. Chem. Soc. 129:4612-4619; Shin et al. (2008) Chem. Mater. 20:239-249; Riccio et al. (2011) Chem. Mater. 23:1727-1735; Carpenter et al. (2012) Biomacromolecules 13(10) 3334-3342)
- NO-releasing dendrimers
   (Stasko et al. (2006) J. Am. Chem. Soc. 128:8265-8271; Stasko et al. (2008) Biomacromolecules. 9:834-841; Lu et al. (2013) Biomacromolecules 14(10) 3589-3598)
- NO-releasing synthetic polymers:
   Mowery KA, Schoenfisch MH, Saavedra JE, Keefer LK, Meyerhoff ME. Preparation and characterization of hydrophobic polymeric films that are thromboresistant via nitric oxide release. Biomaterials. 2000; 21:9-21. [PubMed: 10619674]
   Parzuchowski PG, Frost MC, Meyerhoff ME. Synthesis and Characterization of Polymethacrylate-Based Nitric Oxide Donors. J. Am. Chem. Soc. 2002; 124:12182-12191. [PubMed: 12371858]
   Coneski PN, Rao KS, Schoenfisch MH. Degradable Nitric Oxide-Releasing Biomaterials via Post-Polymerization Functionalization of Cross-Linked Polyesters. Biomacromolecules. 2010; 11:3208-3215.
- NO-releasing electrospun fibers:
   Coneski PN, Nash JA, Schoenfisch MH. Nitric Oxide-Releasing Electrospun Polymer Microfibers. ACS Appl. Mater. Interfaces. 2011; 3:426-432. [PubMed: 21250642]
- metallic clusters, and polymers; self-assembled monolayers, polyurethanes, and polyesters
- N-diazeniumdiolate
- S-nitrosothiols (RSNOs)
- tertiary thiol-based silane precursors
- alkoxy- or alkylalkoxysilanes, e.g., tetramethoxysilane (TMOS), tetraethoxysilane (TE-OS), methyltrimethoxysilane (MTMOS), or isobutyltrimethoxysilane (BTMOS)
- S-nitroso-N-acetyl-DL-penicillamine (SNAP) surface-grafted silica particles, silicone rubber films
- RSNO-modified xerogels using mercaptopropyl- and methyltrimethoxysilane (MPTMS and MTMOS)
- Tertiary thiol silanes
- 3-Aminopropyltrimethoxysilane (APTMS), tetraethoxysilane (TEOS), isobutyltrimethoxysilane (BTMOS), Methyltrimethoxysilane (MTMOS), diethylenetriamine pentaacetic acid (DTPA), Tetramethoxysilane (TMOS), D(-)-Penicillamine, N-(6-aminohexyl) aminopropyl trimethoxysiloxane (DACA-6), potassium tetrakis (4-Chlorophenyl) borate.
- DACA-6 Doped Silicone
- NO-releasing silicone resin oligomers.

If any one of the above NO-releasing materials cannot release NO on its own, the material may be charged with NO or a NO precursor to confer NO-releasing capability to the material.

Particularly suitable NO-releasing compounds and compositions are described in Amoako et al. (2012) ASAIO J. 58(3): 238-246; and in Riccio et al. cited above, the content of which is incorporated herein by reference. The NO-releasing materials disclosed in US 7,811,600 B2, US 7,829,553 B2, US 2009/0232863 A1 and US 2011/0151000 A1 may be used in accordance with the present invention and are incorporated herein by reference.

The filling comprising the NO-releasing material may be prepared as described in Riccio et al. (2012) ACS Appl Mater Interfaces 4(2): 796-804 and Amoako et al. (2012) ASAIO J. 58(3): 238-246.

In one embodiment, the NO-releasing material is uniformly dispersed in said filling. In another embodiment, the NO-releasing material is dissolved in said filling.

The concentration of the NO-releasing material is the filling may range from 1 ppm to 1,000 ppm, preferably from 5 ppm to 100 ppm, most preferably from 10 ppm to 50 ppm (by weight).

Upon implantation, the NO is released from the implant for a period of at least one month, preferably at least three months, more preferably at least 6 months, most preferably at least one year.

The nitric oxide is typically released from the breast implant at a rate of from 0.01 nanomol/(min*cm²) to 100 nanomol/(min*cm²), preferably at a rate of from 0.05 nanomol/(min*cm²) to 10 nanomol/(min*cm²), more preferably at a rate of from 0.1 nanomol/(min*cm²) to 1 nanomol/(min*cm²). The release rate can be determined by the test described in Amoako et al. (2012) ASAIO J. 2012 ; 58(3): 238-246; or in Coneski and Schoefisch (2012) Chem. Soc. Rev. 41(10) 3753-3758.

In certain embodiments, the NO-charged breast implant is able to deliver NO on demand by an external signal (e.g. ultra violet light, ultra sound or another type of trigger).

In one particular embodiment, the release rate of NO from the implant upon implantation is substantially constant. The amount of total NO released over time is substantially linear.

In another embodiment, which may be combined with the other embodiments described herein, the NO-releasing material is not only contained in the filling, but also on or within the shell material. In this embodiment, the shell is coated with an NO-releasing material described hereinabove. The NO-releasing material of the shell may be the same or different from the NO-releasing material contained in the filling.

The shell surface of the breast implant of the invention may be smooth or textured.

The volume of the breast implant may range from 100 ml to 750 ml, preferably from 125 to 650 ml, more preferably from 150 to 500 ml, most preferably from 200 to 400 ml.

The width of the breast implant may range from 8 to 18 cm, preferably from 9 to 16 cm, more preferably from 10 to 15 cm.

The height of the breast implant may range from 8 to 15 cm, preferably from 9 to 14 cm, more preferably from 10 to 13 cm.

The projection of the breast implant may range from 3 to 6 cm, preferably from 3.1 to 5.7 cm, more preferably from 3.5 to 5 cm.

A further aspect of this invention is the use of a breast implant comprising a nitric oxide releasing material in a method of breast augmentation, wherein said method comprises implanting the breast implant into a patient's breast, wherein said patient is resistant to at least one antibiotic. A further aspect of this invention is the use of a breast implant comprising a nitric oxide releasing material in a method of breast augmentation, wherein said method comprises implanting the breast implant into a patient's breast, wherein said patient is allergic to at least one antibiotic. Yet another aspect of the invention the use of a breast implant comprising a nitric oxide releasing material in a method of breast augmentation, wherein said method comprises implanting the breast implant into a patient's breast, wherein no antibiotics are administered to the patient within 1 week upon implantation. Yet another aspect of the invention is the use of a breast implant comprising a nitric oxide releasing material in a method of breast augmentation, wherein said method comprises implanting the breast implant into a patient's breast without administering antibiotics to the patient. Yet another aspect of the invention is the use of a breast implant comprising a nitric oxide releasing material in a method of breast augmentation, wherein said method comprises implanting the breast implant into a patient's breast wherein said patient has multi drug resistance (MDR).

Many plastic surgeons administer antibiotics to the patient during and after breast implantation to reduce the risk of bacterial infection. It has been found by the inventors that this is not necessary when the breast implant comprises a NO-releasing material which is capable of releasing NO upon implantation. The NO-releasing material may be coated onto the shell, and/or it may be contained within the filling of the breast implant as described above. This avoids side effects associated with administration of antibiotics, such as the intolerance or the development of resistance to antibiotics. In addition, the use of antibiotics causes significant costs.

In particular, the present invention allows treating patients which - due to their allergy or resistance to antibiotics - could be treated only with a much higher risk of infection, if at all.

The use of the breast implant of the invention further reduces the risk that a later surgical intervention becomes necessary in the case of capsular contracture, as nitric oxide reduces fibrosis and the formation of scars.

Yet another aspect of the invention is the use of a breast implant as defined herein in a method of breast augmentation. The breast augmentation may be primary augmentation (women who received breast implants to increase the size or change the shape of their breasts), primary reconstruction (women who received breast implants to replace breast tissue that was removed due to disease or trauma or that failed to develop properly), revision augmentation (women who received breast implants to correct or improve the results of primary breast augmentation surgeries) or revision reconstruction (women who received breast implants to correct or improve the results of primary breast reconstruction surgeries).

Yet another aspect of the invention is the use of a breast implant as defined herein for use in a method of preventing bacterial infection during or after plastic surgery. Yet another aspect of the invention is the use of a breast implant as defined herein for use in a method of preventing or treating inflammation during or after plastic surgery.

A further aspect of the invention is the use of a breast implant as defined herein for preventing the formation of capsular contracture upon breast augmentation surgery. Capsular contracture is the hardening of the breast area around the implant. It can occur in the tissue surrounding one or both implants. This hardening causes the tissue to tighten, which can be painful. There are four grades of capsular contracture, known as Baker Grades:

| | |
|---|---|
| Grade I | Breast is normally soft and looks natural |
| Grade II | Breast is a little firm but looks normal |
| Grade III | Breast is firm and looks abnormal |
| Grade IV | Breast is hard, painful, and looks abnormal |

Grades III and IV capsular contracture are considered severe, and may require reoperation or implant removal. Capsular contracture, whether mild, moderate or severe, may occur more than once in the same implant.

In a first embodiment, the breast implant of the invention is used to prevent capsular contracture Grade I. In another embodiment, the breast implant of the invention is used to prevent capsular contracture Grade II. In another embodiment, the breast implant of the invention is used to prevent capsular contracture Grade III. In yet another embodiment, the breast implant of the invention is used to prevent capsular contracture Grade IV.

## Claims

1. A breast implant comprising a flexible shell having a non-solid filling, said filling comprising a nitric oxide releasing material, wherein said flexible shell is permeable for nitric oxide.

2. The breast implant of claim 1, wherein said filling further comprises a silicone gel.

3. The breast implant of claim 1 or 2, wherein the nitric oxide releasing material is uniformly dispersed in said filling.

4. The breast implant of any one of the preceding claims, wherein the nitric oxide releasing material comprises a compound selected from the group consisting of organic nitrates, organic nitrites, metal-NO-complexes, N-nitrosamines, N-hydroxyl nitrosamines, nitrosimines, nitrosothiols, C-nitroso compounds, diazetine dioxides, furoxans, benzofuroxans, oxatriazole-5-imines, syndonimines, oxims, hydroxyamines, guanidines, N-hydroxyguanidines, hydroxyurea, hydroxamic acids, and nitroarenes.

5. The breast implant of any one of the preceding claims, wherein the nitric oxide is released from the breast implant for a period of at least one month, preferably for at least one year.

6. The breast implant of any one of the preceding claims, wherein the nitric oxide is released from the breast implant at a rate from 1*10⁻¹² mole*min⁻¹*cm⁻² to 1*10⁻¹⁰ mole*min¹*cm⁻².

7. The breast implant of any one of the preceding claims, wherein the flexible shell comprises a polysiloxane.

8. The breast implant of any one of the preceding claims, wherein the flexible shell comprises or consists of a material selected from the group consisting of polydimethylsiloxane, polydiphenylsiloxane, and combinations thereof.

9. The breast implant of any one of the preceding claims, wherein the flexible shell is coated with a nitric oxide releasing material.

10. A breast implant comprising a nitric oxide releasing material for use in a method of breast augmentation, wherein said method comprises implanting the breast implant into a patient's breast, wherein said patient is resistant and/or allergic to at least one antibiotic.

11. A breast implant comprising a nitric oxide releasing material for use in a method of breast augmentation, wherein said method comprises implanting the breast implant into a patient's breast without administering antibiotics to the patient.

12. The breast implant for use according to claim 10 or 11, wherein said breast implant is a breast implant as defined in any one of claims 1 to 9.

13. A breast implant as defined in any one of claims 1 to 9 for use in a method of breast augmentation.

14. A breast implant as defined in any one of claims 1 to 9 for use in a method of preventing bacterial infection during or after plastic surgery.

15. A breast implant as defined in any one of claims 1 to 9 for preventing the formation of capsular contracture upon breast augmentation surgery.
